# EUROPÄISCHE PATENTSCHRIFT

(11) **EP 2 099 380 B1**
(45) Veröffentlichungstag und Bekanntmachung des Hinweises auf die Patenterteilung: **12.05.2010**
(21) Anmeldenummer: 08852902.9
(22) Anmeldetag: 18.11.2008
(51) Int. Cl.: A61B 19/00

(54) **VERFAHREN ZUM ERMITTELN EINES AUFSTELLORTES UND ZUM AUFSTELLEN EINER ERFASSUNGSVORRICHTUNG EINES NAVIGATIONSSYSTEMS**
METHOD FOR DETERMINING A POSITION FOR AND POSITIONING A DETECTION DEVICE OF A NAVIGATION SYSTEM
PROCÉDÉ POUR DÉTERMINER UN EMPLACEMENT D'INSTALLATION D'UN DISPOSITIF DE DÉTECTION D'UN SYSTÈME DE NAVIGATION ET POUR INSTALLER CE DISPOSITIF

(30) Priorität: 19.11.2007 DE 102007055205
(43) Veröffentlichungstag der Anmeldung: 16.09.2009
(73) Patentinhaber: KUKA Roboter GmbH, 86165 Augsburg (DE)
(72) Erfinder: ORTMAIER, Tobias, 82110 Germering (DE); JACOB, Dirk, 86399 Bobingen (DE)
(74) Vertreter: Patentanwälte Funk & Böss GbR
(86) Internationale Anmeldenummer: PCT/EP2008/065756
(87) Internationale Veröffentlichungsnummer: WO 2009/065830

(56) Entgegenhaltungen:
- EP-A- 1 340 470
- WO-A-2007/069170
- US-A1- 2004 116 803
- US-B1- 6 366 799

## Beschreibung

Die Erfindung betrifft ein Verfahren zum Ermitteln eines Aufstellortes einer Erfassungsvorrichtung eines Navigationssystems und ein Verfahren zum Aufstellen einer Erfassungsvorrichtung eines Navigationssystems.

Die US 2004/0015053 A1 offenbart einen medizinischen Arbeitplatz mit einem Laparoskop, einem das Laparoskop führenden Roboter und weiteren chirurgischen Instrumenten. Am Laparoskop und an den chirurgischen Instrumenten sind Positionssensoren angeordnet, die ein von einem Emitter eines Navigationssystems gesendetes elektromagnetisches Feld detektieren. Aufgrund des detektierten elektromagnetischen Feldes können die Positionen des Laparoskops und der chirurgischen Instrumente im Raum ermittelt werden.

Die US 2004/0077939 A1 offenbart einen medizinischen Arbeitsplatz mit einem Röntgengerät, einem chirurgischen Instrument, einem Positionserfassungssystem und einem das chirurgische Instrument führenden Roboter zur Behandlung eines Patienten in zumindest teilweise automatisierter Weise. Um die Positionen des chirurgischen Instruments, des Röntgengerätes und des Patienten zu erfassen, sind am Röntgengerät, am Patienten und am chirurgischen Instrument oder am Roboter Positionsmarker angeordnet, die von einer optischen Positionserfassungsvorrichtung des Positionserfassungssystems aufgenommen werden. Aufgrund einer Auswertung der mit der optischen Positionserfassungsvorrichtung aufgenommenen Bilder der Positionsmarker kann die Position, d.h. die Lage und Orientierung der Positionsmarker und somit des chirurgischen Instruments, des Röntgengerätes und des Patienten im Raum ermittelt werden.

Um die entsprechenden Positionen ermitteln zu können, ist es nötig, dass die Positionserfassungsvorrichtung des Positionserfassungssystems, das auch als Navigationssystem bezeichnet wird, die Positionsmarker ausreichend gut erfassen kann. Handelt es sich bei dem Navigationssystem um ein optisches Navigationssystem, dessen Positionserfassungsvorrichtung z.B. als Kameras ausgeführt ist, dann kann beispielsweise die Qualität der Positionsermittlung leiden, wenn die Positionsmarker relativ zu den Kameras auch nur teilweise verdeckt sind.
US 6366799 offenbart ein Verfahren zum verbesserten Aufstellen eines Navigationssystems.

Die Aufgabe der Erfindung ist es daher, ein Verfahren zum Ermitteln eines Aufstellortes einer Erfassungsvorrichtung eines Navigationssystems anzugeben, mit dessen Hilfe Aufstellorte für die Erfassungsvorrichtung relativ zu einem Roboter und/oder einem dreidimensionalen Objekt gefunden werden können, sodass die Position des Roboters und/oder des Objekts mittels der Erfassungsvorrichtung relativ genau ermittelt werden kann.

Eine weitere Aufgabe der Erfindung ist es, ein Verfahren zum Aufstellen einer Erfassungsvorrichtung eines Navigationssystems relativ zu einem Roboter und/oder einem Objekt anzugeben, sodass mittels der Erfassungsvorrichtung die Position des Roboters und/oder des dreidimensionalen Objekts relativ genau ermittelt werden kann.

Die erste Aufgabe der Erfindung wird gelöst durch ein Verfahren zum Ermitteln eines Aufstellortes einer Erfassungsvorrichtung eines Navigationssystems, aufweisend folgende Verfahrensschritte:
- Erstellen einer Rechner-Simulation eines Systems, das
   - eine Erfassungsvorrichtung eines Navigationssystems,
   - einen mit ersten Markern des Navigationssystems versehenen Roboter oder mit ersten markanten Stellen versehenen Roboter, und
   - ein mit zweiten Markern des Navigationssystems versehenes dreidimensionales Objekt oder mit zweiten markanten Stellen versehenes Objekt
   aufweist,
- Simulieren verschiedener Aufstellorte des Roboters, des Objekts und/oder der Erfassungsvorrichtung mittels der Rechner-Simulation,
- automatisches Ermitteln der Qualität der Erfassbarkeit der ersten Marker bzw. der ersten markanten Stellen des Roboters und/oder der zweiten Marker bzw. der zweiten markanten Stellen des Objekts mittels der Erfassungsvorrichtung für die simulierten Aufstellorte, und
- Ausgeben der ermittelten Qualitäten und die entsprechenden simulierten Aufstellorte und/oder des simulierten Aufstellorts mit der höchsten oder zumindest mit einer ausreichend hohen ermittelten Qualität.

Somit ist es möglich, automatische oder interaktiv die Aufstellorte des Roboters, des Objekts und/oder der Erfassungsvorrichtung anzupassen, so dass eine ausreichend gute Qualität erreicht wird.

Navigationssysteme sind in der Medizintechnik, insbesondere in der minimalinvasiven Medizintechnik, beispielsweise aus der US 6,895,268 B1 allgemein bekannt. Navigationssysteme umfassen die Erfassungsvorrichtung, die beispielsweise eine optische Erfassungsvorrichtung ist, die z.B. Kameras, ein Lasertrackingsystem, Projektoren für ein strukturiertes Licht oder Linienprojektoren aufweisen kann. Die Erfassungsvorrichtung ist eingerichtet, in allgemein bekannter Weise die am Objekt, insbesondere an der Oberfläche des Objekts angeordneten zweiten Marker bzw. die zweiten markanten Stellen des Objekts und die am Roboter angeordneten ersten Marker bzw. die ersten markanten Stellen des Roboters zu erfassen. Aufgrund der mit der Erfassungsvorrichtung erfassten Marker bzw. markanter Stellen kann eine Rechenvorrichtung des Navigationssystems in im Wesentlichen allgemein bekannter Weise die Positionen und gegebenenfalls die Orientierungen des Objekts und des Roboters bestimmen.

Um insbesondere die Aufstellorte des Objekts relativ zur Erfassungsvorrichtung und/oder des Roboters relativ zur Erfassungsvorrichtung zu planen, wird erfindungsgemäß zunächst die Rechen-Simulation für das System aus Roboter mit ersten Markern bzw. ersten markanten Stellen, Objekt mit zweiten Markern bzw., zweiten markanten Stellen und Erfassungsvorrichtung des Navigationssystems erstellt.

Für die Rechen-Simulation kann z.B. ein Bilddatensatz, insbesondere ein dreidimensionaler Bilddatensatz vom Objekt, das beispielsweise ein Lebewesen ist, erstellt werden. Im Bilddatensatz ist das Objekt, gegebenenfalls das Lebewesen, und die zweiten Marker bzw. die zweiten markanten Stellen des Objekts abgebildet.

Die Erfassungsvorrichtung ist ebenfalls für die Rechen-Simulation modelliert, sodass mittels der Simulation Aufstellorte der Erfassungsvorrichtung relativ zum Objekt simuliert werden können. Aufgrund dieser Simulation kann die Position und gegebenenfalls die Orientierung der zweiten Marker bzw. der zweiten markanten Stellen relativ zur Erfassungsvorrichtung ermittelt werden, wodurch die Qualität der Erfassbarkeit der zweiten Marker bzw. der zweiten markanten Stellen mittels der Erfassungsvorrichtung ermittelbar ist. Aufgrund verschiedener simulierter Aufstellorte der Erfassungsvorrichtung relativ zum Objekt kann somit die Qualität der verschiedenen simulierten Aufstellorte berechnet werden. Anschließend ist es möglich, z.B. den Aufstellort der Erfassungsvorrichtung relativ zum Objekt auszugeben, beispielsweise mittels einer Anzeigevorrichtung anzuzeigen, der die beste Qualität der Erfassung ergibt, oder den Ort der Erfassungsvorrichtung automatisch anzupassen.

Handelt es sich bei dem Objekt um das Lebewesen, dann kann der Bilddatensatze vom Lebewesen mittels eines bildgebenden medizintechnischen Gerätes erstellt werden. Geeignete bildgebende medizintechnische Geräte sind z.B. Magnetresonanzgeräte, Computertomographiegeräte, Röntgengeräte, insbesondere C-Bogen Röntgengeräte, oder Ultraschallgeräte.

Der Roboter mit den ersten Markern bzw. mit den ersten markanten Stellen kann ebenfalls für die Rechen-Simulation modelliert werden, sodass auch hier die Erfassbarkeit der am Roboter angeordneten ersten Marker bzw. ersten markanten Stellen für verschiedene Aufstellorte des Roboters relativ zur Erfassungsvorrichtung simuliert und dadurch günstige Aufstellorte ermittelt werden können. Insbesondere kann der Eingriff oder Teile davon und somit die Bewegung der ersten Marker bzw. der ersten markanten Stellen simuliert werden. Die Bewegung der ersten Marker bzw. der ersten markanten Stellen kann dann zur Berechnung des Aufstellortes herangezogen werden.

Handelt es sich bei dem Objekt um das Lebewesen, das z.B. auf einer Patientenliege liegen soll, dann kann für die Rechen-Simulation auch die Patientenliege modelliert werden, um z.B. zu erkennen, ob die Patientenliege für einen bestimmte Aufstellort die ersten oder zweiten Marker bzw. die ersten oder zweiten markanten Stellen bezüglich der Erfassungsvorrichtung verdeckt oder ob das auf der Patientenliege liegende Lebewesen die Marker bzw. markanten Stellen je nach Ausrichtung der Patientenliege, die z.B. höhenverstellbar ist, verdeckt.

Ist es vorgesehen, den Roboter zu bewegen, dann kann die Bewegung des Roboters für die Rechner-Simulation berücksichtigt werden. Für die Bewegung kann insbesondere die Kinematik des Roboters für die Rechner-Simulation berücksichtigt werden.

Roboter im Allgemeinen sind Handhabungsmaschinen, die zur selbsttätigen Handhabung von Objekten mit zweckdienlichen Werkzeugen ausgerüstet und bezüglich mehrerer Bewegungsachsen, insbesondere hinsichtlich Orientierung, Position und Arbeitsablauf programmierbar sind. Roboter umfassen im Allgemeinen einen Roboterarm, der auch als Manipulator bezeichnet wird, eine Steuervorrichtung und gegebenenfalls einen Effektor, der zum Beispiel als ein Greifer zum Greifen eines Werkzeugs oder, wenn zum Beispiel in der Medizintechnik angewandt, zum Anbringen eines medizinischen Instruments, insbesondere eines chirurgischen Instruments, ausgebildet sein kann. Der Roboterarm stellt im Wesentlichen den beweglichen Teil des Roboters, der auch als Kinematik bezeichnet wird, dar. Der Roboterarm weist insbesondere mehrere Achsen auf, die zum Beispiel mittels elektrischer Antriebe von der zum Beispiel als Rechner ausgeführten Steuervorrichtung angesteuert werden.

Wird der Roboter im medizinischen Umfeld verwendet, dann ist es gemäß einer Ausführungsform des erfindungsgemäßen Verfahrens vorgesehen, dass der Roboter automatisch oder z.B. durch einen Chirurgen ferngesteuert ein medizinisches Instrument bewegt, um das als Lebewesen ausgebildete Objekt damit zu behandeln. Ein medizinisches Instrument ist z.B. ein Endoskop, mit dem das Lebewesen behandelt werden soll.

Somit ist es nach einer Variante des erfindungsgemäßen Verfahrens möglich, eine Bewegung des Roboters aufgrund einer gewollten Bewegung des medizinischen Instruments für das Behandeln des Lebewesens im Rahmen der Rechner-Simulation zu simulieren.

Insbesondere wenn das erfindungsgemäße Verfahren im medizinischen Umfeld verwendet wird, ist es möglich, einer Bedienperson bei einer präoperativen Planung einer Operation des als Lebewesen ausgebildeten Objekts Vorschläge für günstige Aufstellorte der Erfassungsvorrichtung des Navigationssystems relativ zum Roboter und/oder zum Lebewesen anzubieten. Auch die Bewegung des Roboters, insbesondere eine geplante Trajektion der Roboterspitze kann mittels des erfindungsgemäßen Verfahrens bereits mitberücksichtigt werden. Wählt die Bedienperson die Aufstellorte selbständig, dann kann ihr aufgrund des erfindungsgemäßen Verfahrens die erwartete Erfassbarkeit, insbesondere die Sichtbarkeit und Güte der Marker bzw. der markanten Stellen, dargestellt werden, wodurch die Bedienperson gegebenenfalls interaktiv eine Anpassung der geplanten Aufstellorte vornehmen kann.

Die zweite Aufgabe der Erfindung wird gelöst durch ein Verfahren zum Aufstellen einer Erfassungsvorrichtung eines Navigationssystems, aufweisend folgende Verfahrensschritte:
- Aufstellen eines mit ersten Markern eines Navigationssystems oder mit ersten markanten Stellen versehenen Roboters und einer Erfassungsvorrichtung des Navigationssystems, das eingerichtet ist, aufgrund von mit der Erfassungsvorrichtung erfasster erster Marker bzw. erfasster erster markanter Stellen die Position des Roboters im Raum zu ermitteln,
- Ermitteln der aktuellen Position des Roboters relativ zur Erfassungsvorrichtung aufgrund des aufgestellten Roboters und der aufgestellten Erfassungsvorrichtung mittels des Navigationssystems,
- automatisches Vergleichen der Position des Roboters relativ zur Erfassungsvorrichtung mit einer vorab simulierten Position des Roboters relativ zur Erfassungsvorrichtung, und
- Ausgeben einer Abweichung zwischen der aktuellen Position des Roboters relativ zur Erfassungsvorrichtung und der simulierten Position des Roboters relativ zur Erfassungsvorrichtung.

Das automatische Vergleichen der Position des Roboters relativ zur Erfassungsvorrichtung kann gegebenenfalls unter Berücksichtigung der aktuellen Konfiguration des Roboters erfolgen.

Die Positionen und Orientierungen der ersten Marker bzw. der ersten markanten Stellen relativ zur Struktur des Roboters sind im Allgemeinen bekannt. Die Positionen und Orientierungen der am Patienten angeordneten zweiten Marker bzw. zweiten markanten Stellen relativ zur Patientenanatomie sind dagegen in der Regel unbekannt, können aber durch einen zusätzlichen, vorgelagerten und allgemein bekannten Schritt bestimmt werden. Dieser Schritt wird üblicherweise als "Registrierung" bezeichnet.

Um eine relativ günstige Position der Erfassungsvorrichtung des Navigationssystems relativ zum Roboter zu ermitteln, in der die Erfassungsvorrichtung relativ gut die ersten Marker bzw. die ersten markanten Stellen des Roboters für die Positionsbestimmung des Roboters aufnehmen kann, wird die Aufstellung des Roboters relativ zur Erfassungsvorrichtung vorab z.B. mittels des obenstehend beschriebenen Verfahrens zum Ermitteln eines Aufstellortes einer Erfassungsvorrichtung eines Navigationssystems geplant.

Die Umsetzung dieser Planung, d.h. die reale Aufstellung der Erfassungsvorrichtung relativ zum Roboter, kann jedoch schwierig sein. Erfindungsgemäß wird daher die reale Aufstellung des Roboters und der Erfassungsvorrichtung mit der geplanten bzw. simulierten Aufstellung automatisch verglichen. Dazu bestimmt z.B. die Erfassungsvorrichtung die reale Position der ersten Marker bzw. der ersten markanten Stellen und somit die reale Position und gegebenenfalls Orientierung des Roboters. Diese wird mit der geplanten bzw. simulierten Position und gegebenenfalls Orientierung verglichen. Ergibt sich eine Abweichung, dann wird diese ausgegeben, indem eine Information über die ermittelte Abweichung z.B. mittels einer Anzeigevorrichtung angezeigt wird.

Aufgrund der ermittelten aktuellen Position und der geplanten bzw. simulierten Position des Roboters ist es möglich, einen der Abweichung entsprechenden Verschiebungsvektor und/oder Rotation zu ermitteln, der dann ausgegeben, insbesondere angezeigt werden kann. Der Verschiebungsvektor bzw. die Rotation kann insbesondere innerhalb der simulierten Position, die z.B. mittels einer Anzeigevorrichtung angezeigt wird, grafisch angezeigt werden.

Nach einer Variante des erfindungsgemäßen Verfahrens wird der Grad der Abweichung ermittelt und dieser z.B. farblich beispielsweise mittels einer Anzeigevorrichtung angezeigt. Somit kann diejenige Person oder können diejenigen Personen, die die Erfassungsvorrichtung relativ zum Roboter aufstellt bzw. aufstellen, durch Variieren der realen Aufstellung und durch Beobachten der entsprechenden Abweichungen denjenigen Aufstellort für die Erfassungsvorrichtung relativ zum Roboter iterativ oder empirisch ermitteln, der dem simulierten Aufstellort für die Erfassungsvorrichtung relativ gut entspricht. Auch ein "Anpointern" des Aufstellorts des Roboters und der Erfassungsvorrichtung z.B. mit einem getrackten Pointer ist denkbar.

Nach einer Ausführungsform des erfindungsgemäßen Verfahrens wird eine gewollten Bewegung des Roboters für die simulierte Position des Roboters berücksichtigt.

Nach einer weiteren Variante des erfindungsgemäßen Verfahrens werden folgende weitere Verfahrensschritte durchgeführt:
- Aufstellen eines mit zweiten Markern oder mit zweiten markanten Stellen versehenen dreidimensionalen Objekts, wobei das Navigationssystem eingerichtet ist, mit der Erfassungsvorrichtung erfasster zweiter Marker die Position des Objekts im Raum zu ermitteln,
- Ermitteln der aktuellen Position des Objekts relativ zur Erfassungsvorrichtung aufgrund des aufgestellten Objekts und der aufgestellten Erfassungsvorrichtung,
- automatisches Vergleichen der Position des Objekts relativ zur Erfassungsvorrichtung mit einer vorab simulierten Position des Objekts relativ zur Erfassungsvorrichtung, und
- Ausgeben einer Abweichung zwischen der aktuellen Position des Objekts relativ zur Erfassungsvorrichtung und der simulierten Position des Objekts relativ zur Erfassungsvorrichtung.

Das dreidimensionale Objekt ist z.B. ein Lebewesen, das insbesondere mittels des Roboters behandelt werden soll. Für diesen Zweck kann der Roboter ein medizinisches Instrument, insbesondere ein chirurgisches Instrument in vorab geplanter Weise führen. Um die Position des Roboters relativ zum Lebewesen, allgemein zum Objekt, zu bestimmen, sind auch am Objekt, z.B. an dessen Oberfläche, Marker, d.h. die zweiten Marker, angeordnet. Während der Behandlung des Lebewesens mit dem Roboter ist es in der Regel wünschenswert, dass die Erfassungsvorrichtung möglichst jederzeit die ersten und zweiten Marker erfassen kann und demnach derart aufgestellt sein soll, dass diese Bedingung stets erfüllt ist. Aufgrund dieser Variante des erfindungsgemäßen Verfahrens ist es möglich, die Erfassungsvorrichtung nicht nur relativ zum Roboter derart auszurichten, dass dessen erste Marker relativ gut von der Erfassungsvorrichtung erfasst werden können, sondern auch die Erfassungsvorrichtung relativ zum Objekt relativ günstig aufzustellen.

Die Positionen und Orientierungen der am Patienten angeordneten zweiten Marker bzw. zweiten markanten Stellen relativ zur Patientenanatomie sind in der Regel unbekannt, können aber durch einen zusätzlichen, vorgelagerten und allgemein bekannten Schritt bestimmt werden. Dieser Schritt wird üblicherweise als "Registrierung" bezeichnet. Die intraoperative Lage der zweiten Marker kann gegebenenfalls von der präoperativen und somit von der Simulation abweichen. Gegebenenfalls kann eine Anpassung für die zu findenden Aufstellorte nach der Registrierung durchgeführt werden.

Ein Ausführungsbeispiel der Erfindung ist exemplarisch in den beigefügten schematischen Zeichnungen dargestellt. Es zeigen:
- Fig. 1: einen Roboter,
- Fig. 2: einen medizinischen Arbeitsplatz aufweisend den Roboter, ein Navigationssystem und eine Patientenliege,
- Fig. 3: ein Flussdiagramm zum Veranschaulichen des Ermittelns von Aufstellorten der Erfassungsvorrichtung des Navigationssystems relativ zum Roboter,
- Fig. 4: ein bildgebendes medizintechnisches Gerät und
- Fig. 5: ein Flussdiagramm zum Veranschaulichen des Aufstellens der Erfassungsvorrichtung des Navigationssystems relativ zum Roboter.

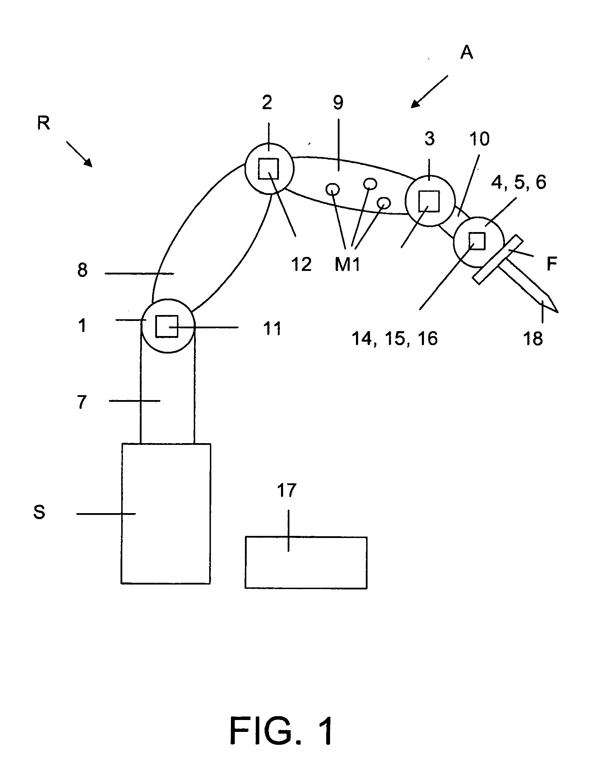

Die Fig. 1 zeigt einen Roboter R mit einem Roboterarm A, der im Falle des vorliegenden Ausführungsbeispieles auf einem Sockel S befestigt ist. Der Roboterarm A stellt im Wesentlichen den beweglichen Teil des Roboters R dar und umfasst mehrere Achsen 1-6, mehrere Hebel 7-10 und einen Flansch F, an dem zum Beispiel ein medizinisches Instrument 18 befestigt werden kann.

Jede der Achsen 1-6 wird im Falle des vorliegenden Ausführungsbeispiels mit einem elektrischen Antrieb 11-16 bewegt, die in nicht dargestellter Weise mit einem Steuerrechner 17 des Roboters R elektrisch verbunden sind, so dass der Steuerrechner 17 bzw. ein auf dem Steuerrechner 17 laufendes Rechnerprogramm die elektrischen Antriebe 11-16 derart ansteuern kann, dass die Position und Orientierung des Flansches F des Roboters R im Wesentlichen frei im Raum eingestellt werden können.

Die elektrischen Antriebe 11-16 des Roboters R umfassen beispielsweise jeweils einen elektrischen Motor und gegebenenfalls eine die Motoren ansteuernde Leistungselektronik.

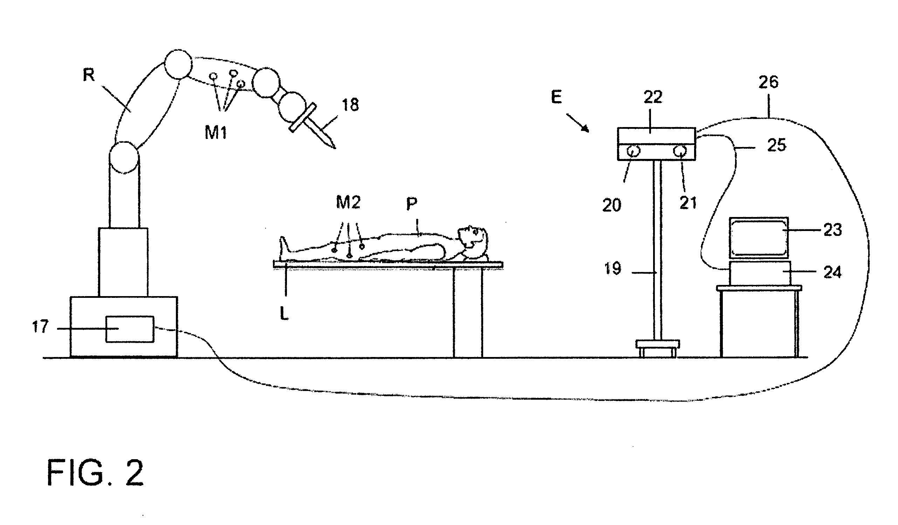

Der Roboter R ist im Falle des vorliegenden Ausführungsbeispiels vorgesehen, einen in der Fig. 2 auf einer Patientenliege L liegenden Patienten P mit dem medizinischen Instrument 18 zu behandeln. Die Fig. 2 zeigt ferner ein Navigationssystem, das eine im Falle des vorliegenden Ausführungsbeispieles zwei Kameras 20, 21 aufweisende Erfassungsvorrichtung E, am Roboter R angeordnete Marker M1 und am Patienten P angeordnete Marker M2 aufweist. Im Falle des vorliegenden Ausführungsbeispiels umfasst die Erfassungsvorrichtung E des Navigationssystems ferner einen Rechner 22 und ist auf einem beweglichen Stativ 19 befestigt, so dass der Aufstellort der Erfassungsvorrichtung E im Raum geändert werden kann.

Navigationssysteme als solche sind dem Fachmann unter Anderem aus der US 6,895,268 B1 bekannt und sind dafür vorgesehen, die Lage und die Orientierung eines Objekts, beispielsweise des Patienten P, zu bestimmen.

Navigationssysteme können beispielsweise magnetische oder, wie es im Falle des vorliegenden Ausführungsbeispiels der Fall ist, optische Navigationssysteme sein und werden beispielsweise dafür eingesetzt, die Position und gegebenenfalls die Orientierung eines Objekts zu ermitteln. Um die Position beispielsweise des Patienten P oder des Roboters R zu ermitteln, ermittelt das Navigationssystem mittels seiner Kamera 20, 21 die Positionen der Marker M1, M2 im Raum.

Aufgrund der ermittelten Positionen des Patienten P und des Roboters R kann dieser z.B. in gewünschter Weise das medizinische Instrument 18 derart bewegen, dass der Patient P in gewünschter Weise damit behandelt wird. Damit der Steuerrechner 17 des Roboters R eine Information über die aktuelle Position des Patienten P erhält, ist beispielsweise der Rechner 22 des Navigationssystems mit dem Steuerrechner 17 des Roboters R mittels einer Datenleitung 26 verbunden.

Damit das Navigationssystem die Positionen des Patienten P und des Roboters R ausreichend genau ermitteln kann, ist es nötig, dass die Kameras 20, 21 die Marker M1, M2 ausreichend gut erfassen können. Somit ist es notwendig, dass die Erfassungsvorrichtung E in geeigneter Weise relativ zum Patienten P und dem Roboter R aufgestellt ist.

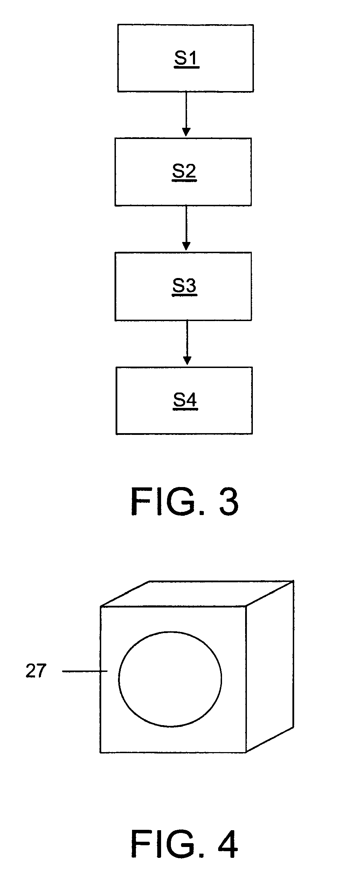

Um zumindest einen relativ günstigen Aufstellort für die Erfassungsvorrichtung E relativ zum Patienten P und relativ zum Roboter R zu erhalten, werden im Falle des vorliegenden Ausführungsbeispiels der Aufstellort des Roboters R, die Lage des Patienten P und der Aufstellort der Erfassungsvorrichtung E geplant. Die im Falle des vorliegenden Ausführungsbeispiels durchgeführten Schritte für die Planung der besagten Aufstellorte sind in einem in der Fig. 3 gezeigten Flussdiagramm zusammengefasst.

Im Falle des vorliegenden Ausführungsbeispiels werden die Aufstellorte der Erfassungsvorrichtung E und des Roboters R, sowie die Lage des Patienten P mittels einer Rechner-Simulation geplant, die in Form eines geeigneten Rechnerprogramm auf einem Rechner 24 gespeichert ist, Schritt S1 des Flussdiagramms der Fig. 3.

Im Falle des vorliegenden Ausführungsbeispiels wird für die Rechner-Simulation zunächst vom Patienten P mittels eines in der Fig. 4 dargestellten medizintechnischen Gerätes 27 ein insbesondere dreidimensionaler Bilddatensatz erzeugt. Das bildgebende medizintechnische Gerät 27 ist beispielsweise ein Magnetresonanzgerät, ein Computertomographiegerät, ein Röntgengerät, insbesondere ein C-Bogen Röntgengerät, oder ein Ultraschallgerät. Für die Erstellung des Bilddatensatzes können die Marker M2 bereits am Patienten P angeordnet sein, die demnach auch in dem dem Bilddatensatz zugeordneten Bild abgebildet sein können. Der dreidimensionale Bilddatensatz wird in den Rechner 24 geladen, wodurch die Rechner-Simulation den Patienten P modellieren kann.

Im Falle des vorliegenden Ausführungsbeispiels sind im Rechner 24 auch Modelle des Roboters R und der Kameras 20, 21 gespeichert. Das Modell des Roboters R umfasst außerdem die am Roboter R angeordneten Marker M1. Somit ist es möglich, verschiedene Aufstellorte des Roboters R und der Erfassungsvorrichtung E und Positionen und/oder Orientierungen des Patienten P mittels der Rechner-Simulation zu simulieren.

Um eine möglichst gute Position der Erfassungsvorrichtung E relativ zum Roboter R und zum Patienten P zu erhalten, simuliert eine in den Figuren nicht näher dargestellte Bedienperson verschiedene Aufstellorte der Erfassungsvorrichtung E, des Roboters R und des Patienten P mittels der Rechner-Simulation, Schritt S2 des Flussdiagramms der Fig. 3.

Im Falle des vorliegenden Ausführungsbeispiels ist die Rechner-Simulation derart ausgeführt, das sie für jeden dieser simulierten Aufstellorte automatisch die Qualität der Erfassbarkeit der ersten und zweiten Marker M1, M2 mittels der Kameras 20, 21 ermittelt, Schritt S3 des Flussdiagramms der Fig. 3. Beispielsweise kann der Abstand der Marker M1, M2 zueinander oder die Verdeckung der Marker M1, M2 untereinander oder mit der Umgebung als Qualitätskriterium herangezogen werden.

Die entsprechend ermittelte Qualität wird anschließend auf einer mit dem Rechner 24 verbundenen Anzeigevorrichtung 23 grafisch dargestellt. Dadurch kann die Bedienperson empirisch oder automatisch eine möglichst gute Position der Kameras 20, 21 bezüglich des Patienten P und des Roboters R ermitteln. Alternativ oder zusätzlich kann die Rechner-Simulation auch derart ausgeführt sein, dass sie z.B. diejenigen simulierten Aufstellorte anzeigt, die die beste oder zumindest hinreichend gute Qualität der Erfassbarkeit ergeben, nachdem mehrere Aufstellorte simuliert wurden, Schritt S4 des Flussdiagramms der Fig. 3.

Nachdem mittels der Rechner-Simulation gewünschte Aufstellorte der Erfassungsvorrichtung E und des Roboters R, sowie die Lage des Patienten P mittels der Rechner-Simulation ermittelt wurden, werden die Erfassungsvorrichtung E, der Roboter R und der Patient P entsprechend der Planung, d.h. der Simulation aufgestellt bzw. auf der Patientenliege L ausgerichtet.

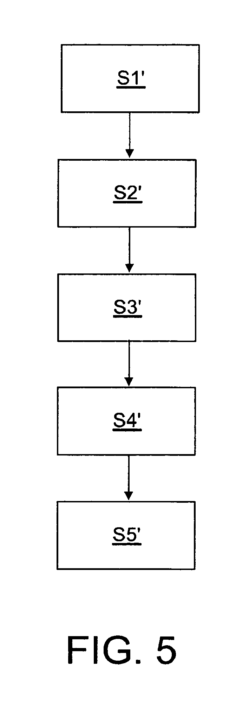

Die Fig. 5 fasst Schritte zusammen, die im Falle des vorliegenden Ausführungsbeispiels durchgeführt werden, damit die Erfassungsvorrichtung E, der Roboter R und der Patient P wie geplant aufgestellt bzw. ausgerichtet werden.

Im Falle des vorliegenden Ausführungsbeispiels läuft auf dem Rechner 24 ein Rechnerprogramm, das die Bedienperson unterstützt, damit diese in relativ einfacher Weise den Roboter R und die Erfassungsvorrichtung E wie geplant aufstellen kann.

Zunächst stellt die Bedienperson den Roboter R und die Erfassungsvorrichtung E auf, Schritt S1' des Flussdiagramms der Fig. 5.

Die Erfassungsvorrichtung E nimmt die Marker M1 des Roboters R auf und berechnet daraufhin die aktuelle Position des Roboters R im Raum bzw. die aktuelle Position des Roboters R relativ zur Erfassungsvorrichtung E, Schritt S2' des Flussdiagramms der Fig. 5.

Dieses Ergebnis wird dem Rechner 24 übermittelt, der mittels einer elektrischen Leitung 25 mit dem Rechner 22 des Navigationssystems verbunden ist. Auf dem Rechner 24 läuft ein Rechnerprogramm, das die aktuelle Position des Roboters R relativ zur Erfassungsvorrichtung E mit der geplanten Position des Roboters R bezüglich zur Erfassungsvorrichtung E vergleicht, Schritt S3' des Flussdiagramms der Fig. 5. Im Falle des vorliegenden Ausführungsbeispiels wurde die geplante Position des Roboters R relativ zur Erfassungsvorrichtung E mittels der obenstehend beschriebenen Rechner-Simulation ermittelt.

Aufgrund der geplanten Position und der aktuellen Position des Roboters R relativ zur Erfassungsvorrichtung E ermittelt das auf dem Rechner 24 laufende Rechnerprogramm eine Abweichung der beiden Positionen, Schritt S4' des Flussdiagramms der Fig. 5, und zeigt auf der Anzeigevorrichtung 23 eine Information über diese Abweichung an, Schritt S5' des Flussdiagramms der Fig. 5.

Im Falle des vorliegenden Ausführungsbeispiels berechnet das auf dem Rechner 24 laufende Rechnerprogramm einen Verschiebungsvektor oder eine Rotation zwischen der geplanten und der aktuellen Positionen des Roboters R relativ zur Erfassungsvorrichtung E und visualisiert diesen auf der Anzeigevorrichtung 23. Der Verschiebungsvektor zeigt z.B. an, in welche Richtung die Erfassungsvorrichtung E verschoben werden muss, damit sie wie geplant aufgestellt ist. Der Verschiebungsvektor wird z.B. in eine angezeigte Darstellung der geplanten Positionen des Roboters R und der Erfassungsvorrichtung E eingeblendet.

Alternativ ist es auch möglich, für die aktuellen Aufstellungen des Roboters R und der Erfassungsvorrichtung E eine Güte der Abweichung von den geplanten Positionen grafisch, beispielsweise mittels verschiedener Farben auf der Anzeigevorrichtung 23 darzustellen.

Im Falle des vorliegenden Ausführungsbeispiels ist es noch vorgesehen, die aktuelle Lage und/oder Ausrichtung des Patienten P relativ zum Roboter R bzw. relativ zur Erfassungsvorrichtung E mit der mittels der Rechner-Simulation geplanten Lage bzw. Ausrichtung des Patienten P abzugleichen.

Zusätzlich ist es noch möglich, eine Bewegung des Roboters R, die dieser für die Behandlung des Patienten P mit dem medizinischen Instrument 18 durchführen soll, in der Rechner-Simulation zu berücksichtigen und diese gegebenenfalls vor der Durchführung der Behandlung mit der Planung abzugleichen.

Es ist auch möglich, die Patientenliege L für die Rechen-Simulation zu berücksichtigen.

Anstelle der ersten und zweiten Marker M1, M2 könne auch geeignete markante Stellen am Roboter R oder am Patienten P verwendet werden.

Obwohl das Ausführungsbeispiel eine Anwendung in der Medizintechnik beschreibt, sind auch Anwendungen der erfindungsgemäßen Verfahren außerhalb der Medizin, zum Beispiel in Messzellen ebenfalls denkbar.

## Patentansprüche

1. Verfahren zum Ermitteln eines Aufstellortes einer Erfassungsvorrichtung eines Navigationssystems, aufweisend folgende Verfahrensschritte:
- Erstellen einer Rechner-Simulation eines Systems, das
- eine Erfassungsvorrichtung (E) eines Navigationssystems,
- einen mit ersten Markern (M1) des Navigationssystems versehenen Roboter (R) oder mit ersten markanten Stellen versehenen Roboter, und
- ein mit zweiten Markern (M2) des Navigationssystems versehenes dreidimensionales Objekt (P) oder mit zweiten markanten Stellen versehenes Objekt
aufweist,
- Simulieren verschiedener Aufstellorte des Roboters (R), des Objekts (P) und/oder der Erfassungsvorrichtung (E) mittels der Rechner-Simulation,
- automatisches Ermitteln der Qualität der Erfassbarkeit der ersten Marker (M1) bzw. der ersten markanten Stellen des Roboters (R) und/oder der zweiten Marker (M2) bzw. der zweiten markanten Stellen des Objekts (P) mittels der Erfassungsvorrichtung (20, 21) für die simulierten Aufstellorte, und
- Ausgeben der ermittelten Qualitäten und die entsprechenden simulierten Aufstellorte und/oder des simulierten Aufstellorts mit der höchsten oder zumindest mit einer ausreichend hohen ermittelten Qualität.

2. Verfahren nach Anspruch 1, aufweisend Berücksichtigen einer Bewegung des Roboters (R) für die Rechner-Simulation insbesondere unter Berücksichtigen der Kinematik des Roboters (R).

3. Verfahren nach Anspruch 1 oder 2, aufweisend Verwenden eines Bilddatensatzes, insbesondere eines dreidimensionalen Bilddatensatzes des Objekts (P) für die Rechner-Simulation, wobei die Lage der zweiten Marker (M2) bzw. der zweiten markanten Stellen berücksichtigt ist.

4. Verfahren nach einem der Ansprüche 1 bis 3, bei dem das Navigationssystem ein optisches Navigationssystem ist, und/oder bei dem das Objekt ein Lebewesen (P) ist und insbesondere der Roboter (R) vorgesehen ist, automatisch ein medizinisches Instrument (18) zu bewegen, um das Lebewesen (P) damit zu behandeln.

5. Verfahren nach Anspruch 3 und 4, aufweisend Erstellen des Bilddatensatzes vom Lebewesen (P) mittels eines bildgebenden medizintechnischen Gerätes (27).

6. Verfahren nach Anspruch 4 oder 5, aufweisend Simulieren einer Bewegung des Roboters (R) aufgrund einer gewollten Bewegung des medizinischen Instruments (18) für das Behandeln des Lebewesens (P) im Rahmen der Rechner-Simulation.

7. Verfahren zum Aufstellen einer Erfassungsvorrichtung eines Navigationssystems, aufweisend folgende Verfahrensschritte:
- Aufstellen eines mit ersten Markern (M1) eines Navigationssystems oder mit ersten markanten Stellen versehenen Roboters (R) und einer Erfassungsvorrichtung (E) des Navigationssystems, das eingerichtet ist, aufgrund von mit der Erfassungsvorrichtung (E) erfasster erster Marker (M1) bzw. erfasster erster markanter Stellen die Position des Roboters (R) im Raum zu ermitteln,
- Ermitteln der aktuellen Position des Roboters (R) relativ zur Erfassungsvorrichtung (E) aufgrund des aufgestellten Roboters (R) und der aufgestellten Erfassungsvorrichtung (E) mittels des Navigationssystems,
- automatisches Vergleichen der Position des Roboters (R) relativ zur Erfassungsvorrichtung (E) mit einer vorab simulierten Position des Roboters (R) relativ zur Erfassungsvorrichtung (E), und
- Ausgeben einer Abweichung zwischen der aktuellen Position des Roboters (R) relativ zur Erfassungsvorrichtung (E) und der simulierten Position des Roboters (R) relativ zur Erfassungsvorrichtung (E).

8. Verfahren nach Anspruch 7, aufweisend Ermitteln der aktuellen Position des Roboters (R) relativ zur Erfassungsvorrichtung (E) mittels Erfassen der am Roboter (R) angeordneten ersten Markern (M1) bzw. der ersten markanten Stellen mittels der Erfassungsvorrichtung (E).

9. Verfahren nach Anspruch 7 oder 8, bei dem die simulierte Position des Roboters (R) relativ zur Erfassungsvorrichtung (E) mittels eines Verfahrens nach einem der Ansprüche 1 bis 6 ermittelt wird.

10. Verfahren nach einem der Ansprüche 7 bis 9, aufweisend
- Ermitteln eines der Abweichung zwischen der aktuellen Position des Roboters (R) relativ zur Erfassungsvorrichtung (E) und der simulierten Position des Roboters (R) relativ zur Erfassungsvorrichtung (E) zugeordneten Verschiebungsvektors und/oder Rotation, und
- Ausgeben des Verschiebungsvektors und oder der Rotation.

11. Verfahren nach einem der Ansprüche 7 bis 10, aufweisend Ermitteln des Grads der Abweichung und Ausgeben des ermittelten Grades.

12. Verfahren nach einem der Ansprüche 7 bis 11, aufweisend Berücksichtigen einer gewollten Bewegung des Roboters (R) für die simulierte Position des Roboters (R).

13. Verfahren nach einem der Ansprüche 7 bis 12, aufweisend
- Aufstellen eines mit zweiten Markern (M2) des Navigationssystems versehenen oder mit zweiten markanten Stellen versehenen dreidimensionalen Objekts (P), wobei das Navigationssystem eingerichtet ist, mit der Erfassungsvorrichtung (E) erfasster zweiter Marker (M2) bzw. erfasster zweiter markanter Stellen, deren Lagen relativ zum Patienten (P) bekannt sind, die Position des Objekts (P) im Raum zu ermitteln,
- Ermitteln der aktuellen Position des Objekts (P) relativ zur Erfassungsvorrichtung (E) aufgrund des aufgestellten Objekts (P) und der aufgestellten Erfassungsvorrichtung (20, 21),
- automatisches Vergleichen der Position des Objekts (P) relativ zur Erfassungsvorrichtung (20, 21) mit einer vorab simulierten Position des Objekts (P) relativ zur Erfassungsvorrichtung (E), und
- Ausgeben einer Abweichung zwischen der aktuellen Position des Objekts (P) relativ zur Erfassungsvorrichtung (E) und der simulierten Position des Objekts (P) relativ zur Erfassungsvorrichtung (E).

14. Verfahren nach Anspruch 13, aufweisend Anpassen einer durch eine Bewegung des Objekts (P) bedingte Änderung der Position (P) und/oder Orientierung der zweiten Marker (M2) bzw. zweiten markanten Stellen relativ zum Objekt (P) für die Abweichung zwischen der aktuellen Position des Objekts (P) relativ zur Erfassungsvorrichtung (E) und der simulierten Position des Objekts (P) relativ zur Erfassungsvorrichtung (E).

15. Verfahren nach Anspruch 13 oder 14, bei dem das Objekt ein Lebewesen (P) ist und insbesondere der Roboter (R) vorgesehen ist, automatisch ein medizinisches Instrument (18) zu bewegen, um das Lebewesen (P) damit zu behandeln.

## Claims

1. A method for determining the placement of a detection device of a navigation system, comprising the following steps:
- preparing of a computer simulation of a system, comprising
- a detection device (E) of a navigation system,
- a robot (R), which is equipped with first markers (M1) of the navigation system or the robot (R), which is equipped with the first prominent points, and
- a three dimensional object (P), which is equipped with second markers (M2) of the navigation system or an object equipped with second prominent points,
- simulating of different placement positions of the robot (R), of the object (P) and/or the detection device (E) by means of the computer simulation,
- automatic determining of the quality of the detection of the first markers (M1), respectively of the first prominent points of the robot (R) and/or the second markers (M2), respectively the second prominent points of the object (P) by means of the detection device (20, 21) for the simulated placement positions, and
- issuing of the determined qualities and the corresponding simulated placement positions and/or of a simulated placement position with the highest or at least with a sufficiently highly determined quality.

2. Method according to claim 1, comprising considering of a movement of the robot (R) for the computer simulation, in particular under consideration of the kinematics of the robot (R).

3. Method according to claim 1 or 2, comprising utilizing of an image data set, in particular of a three dimensional image data set of the object (P) for the computer simulation, wherein the position of the second markers (M2), respectively the second prominent points, have been taken into account.

4. Method according to one of the claims 1 to 3, in which the navigation system is an optical navigation system and/or in which the object is a creature (P) and in particular the robot (R) is designed to automatically move a medical instrument (18) in order to treat the creature (P) with it.

5. Method according to claim 3 and 4, comprising preparing of the image data set of the creature (P) by means of a medical imaging device (27).

6. Method according to claim 4 or 5, comprising simulating of a movement of the robot (R) due to the intentional movement of the medical instrument (18) for the treatment of the creature (P) within the framework of the computer simulation.

7. Method for the positioning of a detection device of a navigation system, comprising the following steps:
- positioning of a robot (R) equipped with first markers (M1) of a navigation system or equipped with first prominent points and of a detection device (E) of a navigation system, which is configured to determine the position of the robot (R) in space due to the detected first markers (M1), respectively the first prominent points,
- determining of the current position of the robot (R) relative to the detection device (E) due to the position of the robot (R) and the positioned detection device (E) by means of the navigation system,
- automatic comparing of the position of the robot (R) relative to the detection device (E) with a preliminarily simulated position of the robot (R) relative to the detection device (E),and
- issuing of a deviation between the current position of the robot (R) relative to the detection device (E) and of the simulated position of the robot (R) relative to the detection device (E).

8. Method according to claim 7, comprising determining of the current position of the robot (R) relative to the detection device (E) by means of detecting of the assigned first markers (M1) on the robot (R), respectively of the first prominent positions by means of the detection device (E).

9. Method according to claim 7 or 8, by which the simulated position of the robot (R) relative to the detection device (E) is determined by means of a method according to one of the clams 1 to 6.

10. Method according to one of the claims 7 to 9, comprising
- determining of the deviation between the current position of the robot (R) relative to the detection device (E) and the simulated position of the robot (R), relative to the detection device (E) and its allocated displacement vector and/or rotation, and
- issuing of the displacement vector and/or the rotation.

11. Method according to one of the claims 7 to 10, comprising determining of the degree of the deviation and issuing of the detected degree.

12. Method according to one of the claims 7 to 11, comprising considering of an intentional movement of the robot (R) for the simulated position of the robot (R).

13. Method according to one of the claims 7 to 12, comprising
- positioning of a three dimensional object (P) equipped with two second markers (M2) of the navigation system or with two prominent points, wherein the navigation system is set up to detect the position of the object (P) in space by means of the second markers (M2), respectively the second prominent points, detected by the detection device (E), which positions and orientation relative to the patient (P) are known,
- determining of the current position of the object (P) relative to the detection device (E) due to the positioning of the object (P) and positioning of the detection device (20, 21),
- automatic comparing of the position of the object (P) relative to the detection device (20, 21) with a preliminarily simulated position of the object (P) relative to the detection device (E), and
- issuing of a deviation between the current position of the object (P) relative to the detection device (E) and of the simulated position of the object (P) relative to the detection device (E).

14. Method according to claim 13, comprising readjustment of a change in the position and/or orientation of the second markers (M2), respectively the second prominent points relative to an object (P), which change is caused by a movement of the object (P), for the deviation between the current position of the object (P) relative to the detection device (E) and of the simulated position of the object (P) relative to the detection device (E).

15. Method according to claim 13 or 14, in which the object is a creature (P) and in particular the robot (R) is designed to automatically move a medical instrument (18) in order to treat the creature (P) with it.

## Revendications

1. Procédé pour déterminer un emplacement d'installation d'un dispositif de détection d'un système de navigation, présentant les étapes de procédé suivantes :
- Etablissement d'une simulation assistée par ordinateur d'un système qui présente
- un dispositif de détection (E) d'un système de navigation,
- un robot (R) pourvu de premiers repères (M1) du système de navigation ou un robot pourvu de premières positions significatives, et
- un objet (P) tridimensionnel pourvu de deux repères (M2) du système de navigation ou un objet pourvu de deux positions significatives,
- Simulation de différents emplacements d'installation du robot (R), de l'objet (P) et/ou du dispositif de détection (E) au moyen de la simulation assistée par ordinateur,
- Détermination automatique de la qualité de la détectabilité des premiers repères (M1) ou des premières positions significatives du robot (R) et/ou des deuxièmes repères (M2) ou des deuxièmes positions significatives de l'objet (P) au moyen du dispositif de détection (20, 21) pour les emplacements d'installation simulés, et
- Affichage des qualités déterminées et des emplacements d'installation simulés correspondants et/ou de l'emplacement d'installation simulé qui présente la plus haute qualité déterminée ou au moins une qualité déterminée suffisamment haute.

2. Procédé selon la revendication 1, présente la prise en compte d'un mouvement du robot (R) pour la simulation assistée par ordinateur, en prenant en particulier en compte la cinématique du robot (R).

3. Procédé selon la revendication 1 ou 2, présentant l'utilisation d'un jeu de données d'image, en particulier d'un jeu de données d'image tridimensionnel de l'objet (P) pour la simulation assistée par ordinateur, la position des deuxièmes repères (M2) ou des deuxièmes positions significatives étant prise en compte.

4. Procédé selon l'une des revendications 1 à 3, dans lequel le système de navigation est un système de navigation optique, et/ou dans lequel l'objet est un être vivant (P) et le robot (R) est prévu pour déplacer automatiquement un instrument médical (18) pour traiter l'être vivant (P) avec celui-ci.

5. Procédé selon les revendications 3 et 4, présentant l'établissement du jeu de données d'image de l'être vivant (P) au moyen d'un appareil technico-médical de visualisation (27).

6. Procédé selon la revendication 4 ou 5, présentant la simulation d'un mouvement du robot (R) en raison d'un mouvement voulu de l'instrument (18) médical pour le traitement de l'être vivant (P) dans le cadre de la simulation assistée par ordinateur.

7. Procédé pour installer un dispositif de détection d'un système de navigation, présentant les étapes de procédé suivantes :
- Installation d'un robot (R) pourvu de premiers repères (M1) ou de premières positions significatives et d'un dispositif de détection (E) du système de navigation qui est aménagé de manière à déterminer la position du robot (R) dans l'espace sur la base de premiers repères (M1) ou de premières positions significatives déterminés avec le dispositif de détection (E),
- Détermination de la position actuelle du robot (R) par rapport au dispositif de détection (E) sur la base du robot (R) installé et du dispositif de détection (E) installé, au moyen du système de navigation,
- Comparaison automatique de la position du robot (R) par rapport au dispositif de détection (E) avec une position simulée auparavant du robot (R) par rapport au dispositif de détection (E), et
- Affichage d'une déviation entre la position actuelle du robot (R) par rapport au dispositif de détection (E) et la position simulée du robot (R) par rapport au dispositif de détection (E).

8. Procédé selon la revendication 7, présentant la détermination de la position actuelle du robot (R) par rapport au dispositif de détection (E) au moyen de la détection des premiers repères (M1) agencés sur le robot (R) ou des premières positions significatives au moyen du dispositif de détection (E).

9. Procédé selon la revendication 7 ou 8, dans lequel la position simulée du robot (R) par rapport au dispositif de détection (E) est déterminée au moyen d'un procédé selon l'une des revendications 1 à 6.

10. Procédé selon l'une des revendications 7 à 9, présentant les étapes suivantes :
- détermination d'un vecteur de translation et/ou d'une rotation associé(e) à la déviation entre la position actuelle du robot (R) par rapport au dispositif de détection (E) et la position simulée du robot (R) par rapport au dispositif de détection (E), et
- affichage du vecteur de translation et/ou de la rotation.

11. Procédé selon l'une des revendications 7 à 10, présentant la détermination du degré de déviation et l'affichage du degré déterminé.

12. Procédé selon l'une des revendications 7 à 11, présentant la prise en compte d'un mouvement voulu du robot (R) pour la position simulée du robot (R).

13. Procédé selon l'une des revendications 7 à 12, présentant les étapes suivantes :
- Installation d'un objet (P) tridimensionnel pourvu de deuxièmes repères (M2) du système de navigation ou de deuxièmes positions significatives, le système de navigation étant aménagé de manière à déterminer la position de l'objet (P) dans l'espace, sur la base de deuxièmes repères (M2) ou de deuxièmes positions significatives détecté(e)s avec le dispositif de détection (E),
- Détermination de la position actuelle de l'objet (P) par rapport au dispositif de détection (E) sur la base de l'objet (P) installé et du dispositif de détection (20, 21) installé,
- Comparaison automatique de la position de l'objet (P) par rapport au dispositif de détection (20, 21) avec une position simulée auparavant de l'objet (P) par rapport au dispositif de détection (E), et
- Affichage d'une déviation entre la position actuelle de l'objet (P) par rapport au dispositif de détection (E) et la position simulée de l'objet (P) par rapport au dispositif de détection (E).

14. Procédé selon la revendication 13, présentant l'adaptation d'une modification de la position (P) due à un mouvement de l'objet (P) et/ou de l'orientation des deuxièmes repères (M2) ou des deuxièmes positions significatives par rapport à l'objet (P) pour la déviation entre la position actuelle de l'objet (P) par rapport au dispositif de détection (E) et la position simulée de l'objet (P) par rapport au dispositif de détection (E).

15. Procédé selon la revendication 13 ou 14, dans lequel l'objet est un être vivant (P) et le robot (R) est en particulier prévu pour déplacer automatiquement un instrument médical (18) pour traiter l'être vivant (P) avec celui-ci.
